(19) Europäisches Patentamt
European Patent Office
Office européen des brevets

(11) **EP 3 305 186 B1**

(12) **FASCICULE DE BREVET EUROPEEN**

(45) Date de publication et mention
de la délivrance du brevet:
**31.05.2023 Bulletin 2023/22**

(21) Numéro de dépôt: **17195257.5**

(22) Date de dépôt: **06.10.2017**

(51) Classification Internationale des Brevets (IPC):
**A61B 5/024** (2006.01)  **A61B 5/0533** (2021.01)
**A61B 5/16** (2006.01)  **A61B 5/00** (2006.01)

(52) Classification Coopérative des Brevets (CPC):
**A61B 5/165; A61B 5/02416; A61B 5/0533;**
**A61B 5/681;** A61B 5/02438; A61B 5/4884;
A61B 5/725

(54) **PROCÉDÉ ET SYSTÈME DE SURVEILLANCE DE STRESS D'UN UTILISATEUR**

VERFAHREN UND SYSTEM ZUR STRESSÜBERWACHUNG EINES BENUTZERS

METHOD AND SYSTEM FOR MONITORING STRESS IN A USER

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **07.10.2016 FR 1659691**

(43) Date de publication de la demande:
**11.04.2018 Bulletin 2018/15**

(73) Titulaire: **Commissariat à l'Energie Atomique et**
**aux Energies**
**Alternatives**
**75015 Paris (FR)**

(72) Inventeurs:
• **OLLANDER, Erik Simon**
**38360 Noyarey (FR)**
• **GODIN, Christelle**
**38190 Villard-Bonnot (FR)**

(74) Mandataire: **Brevalex**
**56, Boulevard de l'Embouchure**
**B.P. 27519**
**31075 Toulouse Cedex 2 (FR)**

(56) Documents cités:
**EP-A1- 2 696 754**   **US-A1- 2016 089 038**
**US-A1- 2016 262 690**

• **ALEXANDRATOS VASILEIOS ET AL: "Mobile**
**real-time arousal detection", 2014 IEEE**
**INTERNATIONAL CONFERENCE ON**
**ACOUSTICS, SPEECH AND SIGNAL**
**PROCESSING (ICASSP), IEEE, 4 mai 2014**
**(2014-05-04), pages 4394-4398, XP032618092,**
**DOI: 10.1109/ICASSP.2014.6854432 [extrait le**
**2014-07-11]**

**Description**

## DOMAINE TECHNIQUE

La présente invention concerne le domaine des procédés et systèmes de surveillance non intrusive de l'état de stress d'une personne et s'applique plus particulièrement à des dispositifs portables de détection de stress.

## ÉTAT DE LA TECHNIQUE ANTÉRIEURE

Le stress est un enjeu majeur de santé publique, en termes de souffrances humaines et coûts économiques. Actuellement, il existe des modèles assez performants pour mesurer le stress d'une personne en utilisant des instruments physiologiques de hautes performances en laboratoire. Toutefois, ce genre d'instrument est encombrant et très difficile à utiliser dans des conditions de la vie courante.

Afin de résoudre ce problème, on commence à utiliser de plus en plus des dispositifs portables permettant de surveiller en continu le stress de la personne portant ce genre de dispositifs. En effet, un dispositif portable permet de surveiller le stress en continu quel que soit l'activité de l'utilisateur, le rendant ainsi intéressant pour des applications de la vie réelle.

Un exemple de ce genre de dispositif est décrit dans le document de Garcia-Mancilla et al., Stress quantification using a wearable device for daily feedback to improve stress management (2016) Lecture Notes in Computer Science, 9545 pp. 204-209. Dans ce document, le stress est caractérisé par une augmentation du rythme cardiaque, une augmentation de la conductance de la peau et un décroissement de la température de la peau. La détection du stress dépend ainsi de trois signaux physiologiques selon un modèle assez complexe qui nécessite un long-terme de détection et une calibration compliquée.

D'autres exemples sont décrits dans les documents US2016/089038, US2016/0262690, et Alexandratos et al. « Mobile real-time arousal détection », ICASSP, 4 mai 2014.

En outre, du fait de la complexité du modèle, les signaux physiologiques sont très sensibles aux mouvements de l'utilisateur, aux pertes de contact, ou à d'autres facteurs environnementaux pouvant engendrer des erreurs de détection.

L'objet de la présente invention est de proposer un procédé et un système de surveillance de stress remédiant aux inconvénients précités, en particulier en fournissant un dispositif portable capable de surveiller en continu le stress d'un utilisateur de manière précise et robuste.

## EXPOSÉ DE L'INVENTION

La présente invention est définie par un procédé non intrusif de surveillance de stress d'un utilisateur, ledit procédé comportant les étapes suivantes :

- mesure en continu d'un rythme cardiaque dudit utilisateur,
- mesure en continu d'une activité électrodermale dudit utilisateur,
- extraction d'une valeur significative de rythme cardiaque sur un intervalle opératoire courant prédéterminé,
- extraction d'une valeur significative d'activité électrodermale sur ledit intervalle opératoire courant, ladite extraction comprenant une détermination d'une valeur caractéristique d'activité électrodermale en comptant un nombre de maxima dudit signal d'activité électrodermale sur ledit intervalle opératoire courant,
- comparaison de ladite valeur significative de rythme cardiaque à un premier seuil prédéterminé,
- comparaison de ladite valeur significative d'activité électrodermale à un deuxième seuil prédéterminé, et
- identification d'un état de stress dudit utilisateur lorsque lesdites valeurs significatives de rythme cardiaque et d'activité électrodermale dépassent simultanément et respectivement les premier et deuxième seuils prédéterminés.

Ce procédé permet d'identifier l'état de stress d'un utilisateur rapidement et de manière robuste en étant très peu sensible aux mouvements de l'utilisateur, aux pertes de contact, ou à d'autres facteurs environnementaux. En effet, le procédé comporte très peu d'étapes et ne dépend que de deux caractéristiques du stress. De plus, un état de stress est confirmé uniquement lorsque les deux caractéristiques sont corrélées augmentant ainsi la robustesse de la détection. Autrement dit, l'état de stress est confirmé lorsque la conjonction des deux caractéristiques est validée.

Avantageusement, l'extraction de ladite valeur significative de rythme cardiaque comporte les étapes suivantes :

- génération d'un signal du rythme cardiaque représentatif de ladite mesure du rythme cardiaque sur ledit intervalle opératoire courant,
- détermination à partir dudit signal du rythme cardiaque d'une valeur de rythme cardiaque moyen sur ledit intervalle opératoire courant,

- détermination de ladite valeur significative de rythme cardiaque en normalisant la valeur de rythme cardiaque moyen par rapport à une valeur moyenne d'un rythme cardiaque d'adaptation et de sa valeur correspondante d'écart-type.

De même, l'extraction de ladite valeur significative d'activité électrodermale comporte :

- une génération d'un signal d'activité électrodermale représentatif de ladite mesure de l'activité électrodermale sur ledit intervalle opératoire courant, et
- une détermination de ladite valeur significative d'activité électrodermale en normalisant ladite valeur caractéristique d'activité électrodermale par rapport à une valeur caractéristique moyenne d'une activité électrodermale d'adaptation et de sa valeur correspondante d'écart-type.

Ceci permet d'adapter la surveillance du stress à l'utilisateur garantissant ainsi plus de précision dans la détection.

Avantageusement, le procédé comporte un filtrage dudit signal d'activité électrodermale avant la détermination de ladite valeur caractéristique d'activité électrodermale.

Ceci permet de supprimer le bruit augmentant davantage la précision et la robustesse de la surveillance du stress.

Avantageusement, la détermination de ladite valeur moyenne de rythme cardiaque d'adaptation et de sa valeur correspondante d'écart-type comporte :

- une mesure d'un rythme cardiaque au repos dudit utilisateur pendant un intervalle de calibration prédéterminé,
- une génération d'un signal de rythme cardiaque au repos sur ledit intervalle de calibration, et
- un calcul de ladite valeur moyenne de rythme cardiaque d'adaptation et de sa valeur correspondante d'écart-type.

De même, la détermination de ladite valeur caractéristique moyenne d'activité électrodermale d'adaptation et de ladite valeur correspondante d'écart-type comporte :

- une mesure d'une activité électrodermale au repos dudit utilisateur pendant ledit intervalle de calibration prédéterminé,
- une génération d'un signal d'activité électrodermale au repos sur ledit intervalle de calibration,
- une détermination de ladite valeur caractéristique d'activité électrodermale d'adaptation sur ledit intervalle de calibration en comptant un nombre de maxima dudit signal d'activité électrodermale au repos,
- un calcul de ladite valeur caractéristique moyenne d'activité électrodermale d'adaptation et de sa valeur correspondante d'écart-type.

Ceci permet d'adapter la surveillance du stress à l'utilisateur de manière rapide et efficace.

Avantageusement, ladite valeur moyenne d'un rythme cardiaque d'adaptation et la valeur correspondante d'écart-type sont par défaut égales à « 0 » et « 1 » respectivement. De même, ladite valeur moyenne d'activité électrodermale d'adaptation et la valeur correspondante d'écart-type sont par défaut égales à « 0 » et « 1 » respectivement.

Ceci permet d'utiliser le procédé de surveillance même si aucune calibration n'est réalisée.

Avantageusement, le procédé comporte une évaluation d'un niveau de stress en fonction des écarts entre d'une part lesdites valeurs significatives de rythme cardiaque et d'activité électrodermale et d'autre part les premier et deuxième seuils prédéterminés.

Ceci permet d'évaluer le niveau de stress avec plus de précision.

Avantageusement, la mesure du rythme cardiaque est réalisée par photo-pléthysmographie.

Cette technique est très adaptée pour des dispositifs portables.

Avantageusement, le premier seuil prédéterminé correspond à une valeur sélectionnée dans un intervalle compris entre 1 et 2. Par ailleurs, le deuxième seuil prédéterminé correspond à une valeur sélectionnée dans un intervalle compris entre 1,5 et 2,5.

Avantageusement, l'intervalle opératoire de temps prédéterminé présente une mesure dont la valeur est comprise entre environ 15 secondes et environ 300 secondes.

Avantageusement, l'intervalle de calibration prédéterminé présente une mesure dont la valeur est plus grande ou égale à environ 300 secondes.

L'invention vise également un système non intrusif de surveillance en continu de l'état de stress d'un utilisateur, comportant :

- un capteur de rythme cardiaque configuré pour mesurer en continu un rythme cardiaque dudit utilisateur,

- un capteur d'activité électrodermale configuré pour mesurer en continu une activité électrodermale dudit utilisateur, et
- des unités de traitement configurées pour :

  - extraire une valeur significative de rythme cardiaque sur un intervalle opératoire courant prédéterminé,

  - extraire une valeur significative d'activité électrodermale sur ledit intervalle opératoire courant, ladite extraction comprenant une détermination d'une valeur caractéristique d'activité électrodermale en comptant un nombre de maxima dudit signal d'activité électrodermale sur ledit intervalle opératoire courant,

  - comparer ladite valeur significative de rythme cardiaque à un premier seuil prédéterminé,
  - comparer ladite valeur significative d'activité électrodermale à un deuxième seuil prédéterminé, et
  - identifier un état de stress dudit utilisateur lorsque lesdites valeurs significatives de rythme cardiaque et d'activité électrodermale dépassent simultanément et respectivement les premier et deuxième seuils prédéterminés.

Avantageusement, les unités de traitement sont configurées pour extraire ladite valeur significative de rythme cardiaque en :

- générant un signal de rythme cardiaque représentatif de ladite mesure du rythme cardiaque sur ledit intervalle opératoire courant,
- déterminant une valeur de rythme cardiaque moyen sur ledit intervalle opératoire courant,
- déterminant ladite valeur significative de rythme cardiaque par normalisation de la valeur de rythme cardiaque moyen par rapport à une valeur moyenne d'un rythme cardiaque d'adaptation et de sa valeur correspondante d'écart-type.

De même, les unités de traitement sont configurées pour extraire ladite valeur significative d'activité électrodermale en :

- générant un signal d'activité électrodermale représentatif de ladite mesure de l'activité électrodermale sur ledit intervalle opératoire courant,
- déterminant une valeur caractéristique d'activité électrodermale par un comptage d'un nombre de maxima dudit signal d'activité électrodermale sur ledit intervalle opératoire courant, et
- déterminant ladite valeur significative d'activité électrodermale par normalisation de ladite valeur caractéristique d'activité électrodermale par rapport à une valeur caractéristique moyenne d'une activité électrodermale d'adaptation et de sa valeur correspondante d'écart-type.

Avantageusement, le système comporte un filtre passe-bas configuré pour filtrer ledit signal d'activité électrodermale.

Avantageusement, le capteur de rythme cardiaque est un capteur photo-pléthysmographie.

Avantageusement, le système comporte une interface indiquant l'état de stress de l'utilisateur.

L'invention vise également un bracelet comportant le système selon l'une quelconque des caractéristiques ci-dessus.

## BRÈVE DESCRIPTION DES DESSINS

La Fig. 1A illustre de manière schématique un système et un procédé de surveillance non intrusive de l'état de stress d'un utilisateur, selon un mode de réalisation de l'invention ;

Les Figs. 1B et 1C illustrent de manière schématique deux modes de réalisation préférés du système de surveillance de la Fig. 1A ;

La Fig. 2 illustre de manière schématique les étapes d'extraction des valeurs significatives de rythme cardiaque et d'activité électrodermale, selon un mode de réalisation de l'invention ;

La Fig. 3 illustre le comptage du nombre de maxima du signal d'activité électrodermale filtré sur un intervalle opératoire, selon un mode de réalisation de l'invention ;

La Fig. 4 illustre de manière schématique un schéma en bloc de surveillance de stress d'un utilisateur, selon un mode de réalisation préféré de l'invention ; et

Les Figs. 5A-5G et 6A-6G sont des exemples de courbes illustrant les étapes de détections de l'état de stress d'un utilisateur, selon l'invention.

**EXPOSÉ DÉTAILLÉ DE MODES DE RÉALISATION PARTICULIERS**

Le principe à la base de l'invention est de proposer un modèle de surveillance de stress basé sur l'acquisition d'un nombre minimal de signaux physiologiques et de l'extraction d'une caractéristique de stress intrinsèquement robuste et discriminante. Ainsi, ce modèle peut être très simplement intégré dans un dispositif portable pour surveiller en continu le stress d'un utilisateur tout en étant très peu sensible aux mouvements de l'utilisateur et aux facteurs environnementaux.

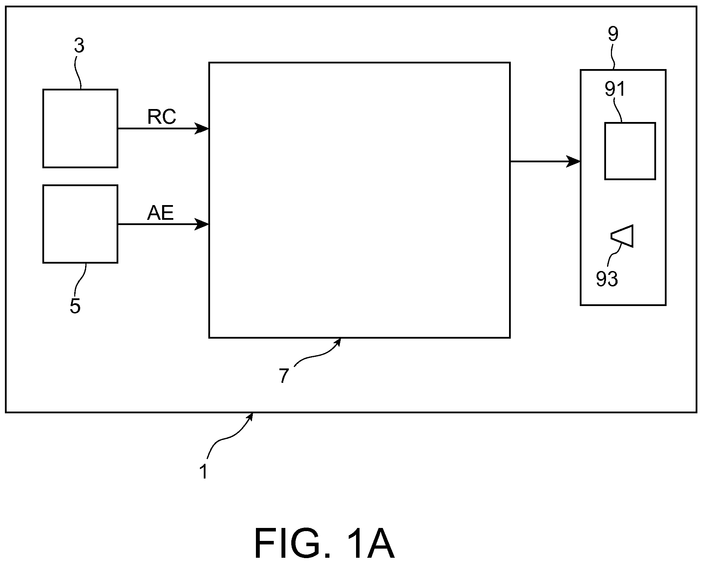

La Fig. 1A illustre de manière schématique un système et un procédé de surveillance non intrusive de l'état de stress d'un utilisateur, selon un mode de réalisation de l'invention.

Ce système de surveillance 1 comporte un capteur 3 de rythme cardiaque, un capteur 5 d'activité électrodermale, des unités de traitement 7 et une interface 9.

Le capteur 3 de rythme cardiaque est configuré pour mesurer en continu le rythme cardiaque de l'utilisateur.

Avantageusement, le capteur 3 de rythme cardiaque est un capteur photopléthysmographe (PPG) très adapté pour des applications ambulatoires. Le capteur PPG (non représenté) comprend des sources lumineuses et des photodiodes correspondantes. Les sources lumineuses envoient de la lumière (par exemple de la lumière verte) vers les veines de l'utilisateur à travers sa peau. La variation de l'intensité de la lumière rouge réfléchie par les veines dépend de la variation du taux d'absorption de la lumière verte incidente qui à son tour dépend de la variation du flux sanguin dans les veines engendrée par le battement du coeur. Ainsi, la variation de l'intensité lumineuse reçue par les photodiodes du capteur PPG permet de déterminer le rythme cardiaque.

En variante, le capteur 3 de rythme cardiaque est un capteur électrocardiographe (ECG). Dans ce cas, le capteur ECG (non représenté) comporte des électrodes qui mesurent des variations d'une tension électrique liée à l'activité cardiaque. Plus particulièrement, l'ECG produit des pics de tension électrique ; la détermination de la distance entre ces pics permet d'identifier les battements cardiaques ainsi que le rythme cardiaque.

Par ailleurs, le capteur 5 d'activité électrodermale est configuré pour mesurer en continu une activité électrodermale (par exemple, une conductance de la peau) de l'utilisateur. Le capteur 5 d'activité électrodermale comporte deux électrodes qui induisent un courant électrique très faible entre deux points de la peau de l'utilisateur et qui mesurent ensuite la conductance électrique entre ces deux points. En effet, la conductance électrique dans la peau dépend de l'état d'activation du système nerveux sympathique de l'utilisateur qui varie en fonction de l'état de stress de ce dernier.

Au cas où les mesures de rythme cardiaque RC et de l'activité électrodermale AE issues des capteurs 3 et 5 sont analogiques, les unités de traitement 7 sont configurées pour les numériser avant d'en extraire des valeurs significatives. En effet, les unités de traitement 7 sont configurées pour extraire à partir des mesures de rythme cardiaque RC une valeur significative de rythme cardiaque sur un intervalle opératoire courant prédéterminé. En outre, les unités de traitement 7 sont configurées pour extraire à partir des mesures d'activité électrodermale AE une valeur significative d'activité électrodermale sur le même intervalle opératoire courant.

On notera que les valeurs significatives de rythme cardiaque et d'activité électrodermale sont avantageusement des valeurs normalisées par rapport à chaque utilisateur. En variante, la normalisation peut être standardisée par défaut quel que soit l'utilisateur.

Les unités de traitement 7 sont ensuite configurées pour comparer la valeur significative de rythme cardiaque à une limite prédéfinie de rythme cardiaque, dite premier seuil prédéterminé. De même, elles sont configurées pour comparer la valeur significative d'activité électrodermale à une limite prédéfinie d'activité électrodermale, dite deuxième seuil prédéterminé.

Grâce à ces comparaisons, les unités de traitement 7 sont configurées pour repérer ou identifier un état de stress de l'utilisateur lorsque les valeurs significatives de rythme cardiaque et d'activité électrodermale dépassent simultanément et respectivement les premier et deuxième seuils prédéterminés.

Par ailleurs, les unités de traitement 7 sont configurées pour envoyer en continu les données concernant l'état de stress de l'utilisateur à l'interface 9. Cette dernière comporte par exemple un écran 91 interactif et un émetteur sonore 93 et est configurée pour indiquer de manière visuelle et/ou sonore l'état de stress de l'utilisateur correspondant aux données reçues depuis les unités de traitement 7.

Avantageusement, les unités de traitement 7 sont configurées pour archiver les données concernant l'état de stress de l'utilisateur ou une partie pertinente de ces données.

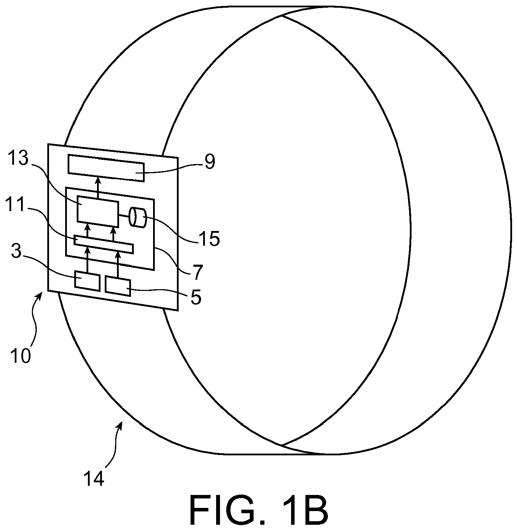

Selon un premier mode de réalisation, le système de surveillance est un dispositif portable autonome. En effet, la Fig. 1B illustre de manière schématique un dispositif portable autonome de surveillance non intrusive de l'état de stress d'un utilisateur, selon ce premier mode préféré de réalisation de l'invention.

Selon ce premier mode de réalisation, le système de surveillance est intégré dans le dispositif portable autonome 10 qui par conséquent comporte un capteur 3 de rythme cardiaque, un capteur 5 d'activité électrodermale, des unités de traitement 7 ainsi qu'une interface 9. A titre d'exemple, le dispositif portable autonome 10 peut être intégré dans une montre à bracelet 14.

Les unités de traitement 7 comportent des unités d'acquisition 11, un microprocesseur 13 et une unité de stockage 15.

Les unités de traitement 7 sont configurées pour récupérer les mesures de rythme cardiaque RC et de l'activité électrodermale AE depuis les capteurs 3 et 5. Eventuellement, si les mesures à la sortie des capteurs sont analogiques, les unités de traitement 7 sont configurées pour numériser ces mesures avant qu'elles soient récupérées par le microprocesseur 13.

Le microprocesseur 13 est configuré pour extraire à partir de ces mesures les valeurs significatives de rythme cardiaque et d'activité électrodermale sur l'intervalle opératoire courant. Il est ensuite configuré pour comparer les valeurs significatives de rythme cardiaque et d'activité électrodermale aux premier et deuxième seuils prédéterminés afin de repérer un état de stress de l'utilisateur lorsque ces valeurs significatives dépassent simultanément et respectivement les premier et deuxième seuils prédéterminés.

Par ailleurs, le microprocesseur 13 est configuré pour commander l'affichage visuel et/ou sonore de l'état de stress de l'utilisateur sur l'interface 9.

Avantageusement, le microprocesseur 13 est configuré pour archiver les données concernant l'état de stress de l'utilisateur ou une partie pertinente de ces données dans l'unité de stockage 15. Le microprocesseur 13 peut éventuellement être configuré pour transmettre en temps réel les données de l'état de stress à un centre de surveillance ou un autre dispositif portable au moyen d'un transmetteur (non représenté).

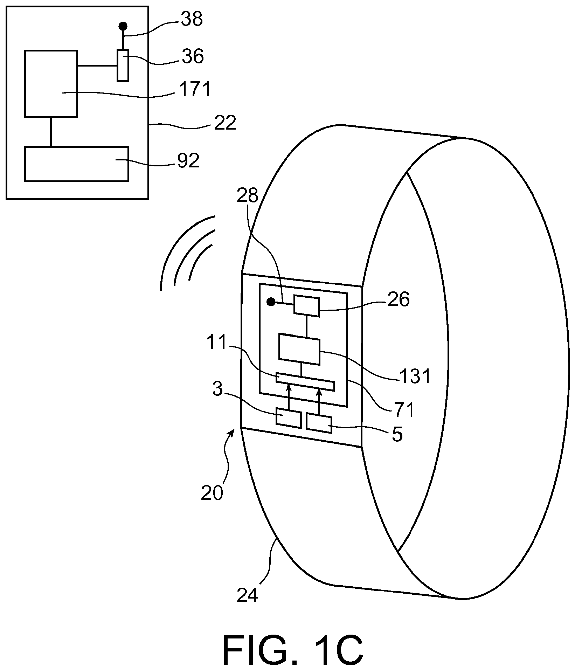

Selon un deuxième mode de réalisation préféré de l'invention, le système de surveillance comporte deux entités séparées: un dispositif de mesure portable 20 et un dispositif de traitement 22 portable (ou non portable) comme illustré de manière schématique sur la Fig. 1C.

Le dispositif de mesure portable 20 est adapté pour être porté par un utilisateur et est destiné à mesurer en continu et de manière non intrusive l'état de stress de l'utilisateur et à transmettre les données de ces mesures au dispositif de traitement 22.

A titre d'exemple, le dispositif de mesure portable 20 est intégré dans un bracelet 24 tandis que le dispositif de traitement 22 peut être un téléphone portable, un ordinateur ou un dispositif de traitement de données quelconque.

Le dispositif de mesure portable 20 comporte un capteur 3 de rythme cardiaque, un capteur 5 d'activité électrodermale et un circuit électronique 71.

Le capteur 3 de rythme cardiaque est configuré pour mesurer en continu le rythme cardiaque de l'utilisateur portant le dispositif portable 20. De même, le capteur 5 d'activité électrodermale est configuré pour mesurer en continu l'activité électrodermale de l'utilisateur.

Le circuit électronique 71 comporte des unités d'acquisition 11, un microprocesseur 131, un premier transmetteur-récepteur 26 et une première antenne 28.

Les unités d'acquisition 11 sont configurées pour récupérer et éventuellement numériser les mesures de rythme cardiaque RC et d'activité électrodermale AE.

Le microprocesseur 131 est configuré pour transformer les signaux numériques des mesures de rythme cardiaque RC et d'activité électrodermale AE en signaux radio.

Le premier transmetteur-récepteur 26 est configuré pour transmettre via la première antenne 28 les signaux radio des mesures de rythme cardiaque RC et d'activité électrodermale AE au dispositif de traitement 22. Le premier transmetteur-récepteur 26 est également configuré pour recevoir des signaux de commande et de contrôle depuis le dispositif de traitement 22. Ces signaux de commande sont traités par le microprocesseur 131 du dispositif portable 20.

Par ailleurs, le dispositif de traitement 22 comporte un deuxième récepteur/transmetteur 36, une deuxième antenne 38, des unités de traitement 171 ainsi que des interfaces de sortie 92.

Le deuxième récepteur-transmetteur 36 est configuré pour recevoir via la deuxième antenne 38 les signaux radio des mesures de rythme cardiaque RC et d'activité électrodermale AE depuis le dispositif portable 20.

Les unités de traitement 171 sont configurées pour extraire à partir de ces signaux des valeurs significatives de rythme cardiaque et d'activité électrodermale sur un intervalle opératoire courant. Elles sont ensuite configurées pour repérer ou identifier un état de stress de l'utilisateur lorsque les valeurs significatives de rythme cardiaque et d'activité électrodermale dépassent simultanément et respectivement des premier et deuxième seuils prédéterminés.

Par ailleurs, les interfaces de sortie 92 sont configurées pour afficher sous la commande des unités de traitement 171 l'état de stress de l'utilisateur de manière visuelle et/ou sonore.

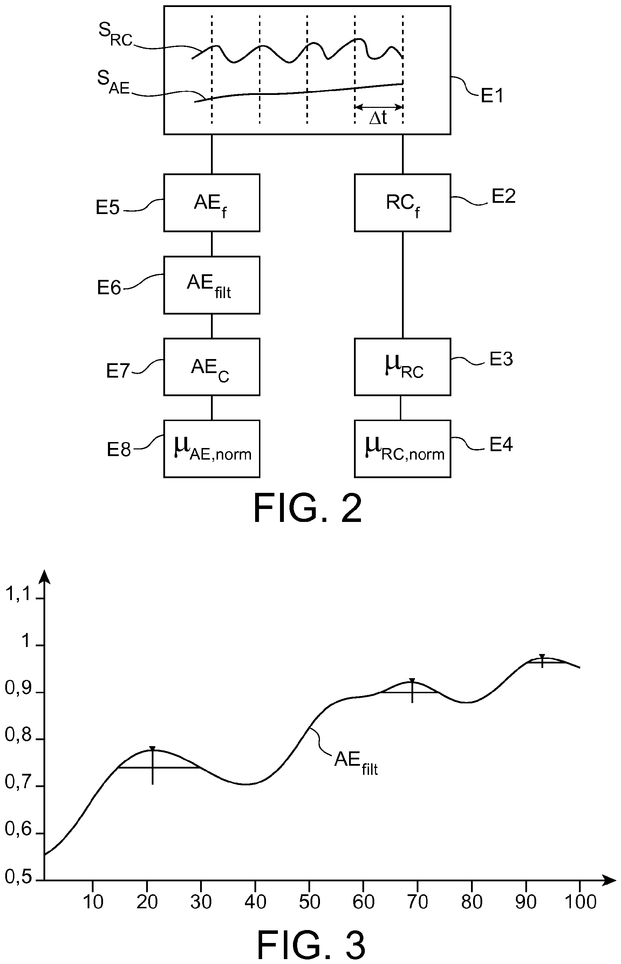

La Fig. 2 illustre de manière schématique les étapes d'extraction des valeurs significatives de rythme cardiaque et d'activité électrodermale, selon un mode de réalisation de l'invention.

Après avoir récupéré les mesures de rythme cardiaque RC et de l'activité électrodermale AE depuis les capteurs 3 et 5, les unités de traitement 7 sont configurées pour générer à l'étape E1 un signal du rythme cardiaque $S_{RC}$ et un signal d'activité électrodermale $S_{AE}$. En outre, les unités de traitement 7 sont configurées pour utiliser un intervalle temporel opératoire $\Delta t$ prédéterminé dont la durée (par exemple 60 secondes) est préenregistrée. Ainsi, le signal courant du rythme cardiaque représentatif des mesures du rythme cardiaque définit les valeurs du rythme cardiaque sur l'intervalle temporel opératoire courant. De même, le signal d'activité électrodermale représentatif des mesures de l'activité électrodermale définit les valeurs de l'activité électrodermale sur l'intervalle temporel opératoire courant.

A l'étape E2, les unités de traitement 7 sont configurées pour échantillonner le signal courant du rythme cardiaque $S_{RC}$ en formant un vecteur $RC_f$ du rythme cardiaque correspondant constitué de N échantillons pour chaque intervalle opératoire $\Delta t$ courant. L'intervalle opératoire $\Delta t$ est ainsi une fenêtre temporelle présentant une mesure (i.e. période) comprise entre environ 15 secondes et environ 300 secondes d'acquisition. Avantageusement, l'intervalle opératoire $\Delta t$ présente une mesure de 60 secondes d'acquisition.

A l'étape E3, les unités de traitement 7 sont configurées pour déterminer une valeur de rythme cardiaque moyen $\mu_{RC}$ pour chaque intervalle opératoire $\Delta t$ courant. Le rythme cardiaque moyen $\mu_{RC}$ est calculé selon la formule suivante :

$$\mu_{RC} = \frac{1}{N} \sum_{n=1}^{N} RC_f(n)$$

avec N le nombre d'échantillons et $RC_f$ le vecteur du rythme cardiaque sur la fenêtre temporelle (intervalle opératoire $\Delta t$ courant).

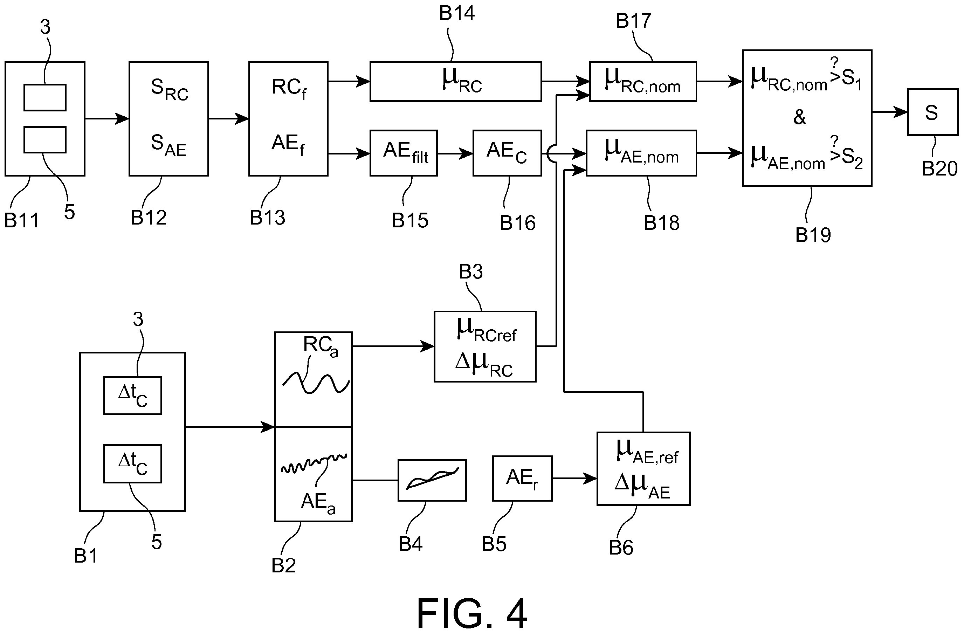

A l'étape E4, les unités de traitement 7 sont configurées pour déterminer la valeur significative de rythme cardiaque $\mu_{RC,norm}$ en normalisant la valeur de rythme cardiaque moyen $\mu_{RC}$ par rapport à une valeur moyenne d'un rythme cardiaque d'adaptation et de sa valeur correspondante d'écart-type (voir l'exemple de la Fig. 4).

A l'étape E5, les unités de traitement 7 sont configurées pour échantillonner le signal courant d'activité électrodermale $S_{AE}$ en formant un vecteur ou signal d'activité électrodermale $AE_f$ constitué de N échantillons pour chaque intervalle opératoire $\Delta t$ courant. Cet intervalle opératoire courant correspond à la même fenêtre temporelle courante que celle correspondant au rythme cardiaque et présente par exemple une mesure d'acquisition de 60 secondes. On notera que la mesure de l'intervalle temporel opératoire $\Delta t$ peut être comprise entre environ 15 secondes et environ 300 secondes.

A l'étape E6, les unités de traitement 7 sont configurées pour filtrer le signal d'activité électrodermale $AE_f$ formant un signal d'activité électrodermale filtré $AE_{filt}$. En effet, les unités de traitement 7 comportent avantageusement un filtre passe-bas de type BUTTERWORTH d'ordre $N_f$ et de fréquence de coupure $f_c$ configuré pour filtrer le signal d'activité électrodermale afin de capter uniquement le comportement phasique de l'activité électrodermale et afin de supprimer le bruit lié aux mesures et acquisitions de données. On notera que la partie phasique de l'activité électrodermale est une bonne représentation des réactions à des événements stressants et cette partie phasique correspond à la dynamique rapide du signal.

Avantageusement, les paramètres $N_f$ et $f_c$ du filtre passe-bas sont sélectionnés parmi les plages de valeurs suivantes :

$$1 \leq N_f \leq 19$$

$$0 < f_c < 2$$

Par exemple, en utilisant un filtre d'ordre $N_f=2$ et de fréquence de coupure $f_c=0,2$Hz, la relation entre l'entrée $AE_f$ du filtre (i.e. le signal courant d'activité électrodermale) et la sortie $AE_{filt}$ du filtre est donnée par l'expression suivante :

$$AE_{filt}[n-2] - 1.561\,AE_{filt}[n-1] + 0.6414\,AE_{filt}[n]$$
$$= 0.0201\,AE_f[n-2] + 0.0402\,AE_f[n-1] + 0.0201\,AE_f[n]$$

A l'étape E7, les unités de traitement 7 sont configurées pour déterminer une valeur caractéristique d'activité électrodermale $AE_c$ en comptant le nombre de maxima du signal d'activité électrodermale filtré $AE_{filt}$ sur l'intervalle opératoire $\Delta t$ courant. Ainsi, la valeur caractéristique d'activité électrodermale $AE_c$ quantifie le nombre de maxima présents dans le signal d'activité électrodermale pendant l'intervalle opératoire courant. En effet, chaque maxima peut être considéré comme une réaction à un événement stressant et représente ainsi une bonne mesure du stress de l'utilisateur.

La Fig. 3 illustre le comptage du nombre de maxima du signal d'activité électrodermale filtré sur un intervalle opératoire courant.

Cette courbe illustre la conductance en $\mu S$ d'un signal d'activité électrodermale filtré $AE_{filt}$ en fonction du temps. La détection de maxima est paramétrée par une hauteur de pic minimal à 0,001 $\mu S$ et une durée minimale de 1 seconde entre deux pics.

Ainsi, une valeur AE$_{filt}$[n] du signal d'activité électrodermale filtré à l'échantillon n est considérée comme un maxima si les conditions suivantes sont vérifiées :

1. Si elle est supérieure aux valeurs voisines :

$$AE_{filt}[n] > AE_{filt}[n+1] \text{ et } AE_{filt}[n] > AE_{filt}[n-1]$$

2. Si aucun maxima n'est détecté dans une durée minimale de 1 seconde autour de l'échantillon n.

Avantageusement, lorsqu'un maxima m est détecté, on ne compte pas les éventuels pics secondaires non significatifs ou erronés qui sont adjacents du pic détecté :

$$AE_{filt}[m] > AE_{filt}[m+1] + 0,001 \text{ et } AE_{filt}[m] > AE_{filt}[m-1] + 0,001$$

La valeur caractéristique d'activité électrodermale AE$_c$ correspond ainsi au nombre de maxima trouvés sur l'intervalle opératoire courant.

A l'étape E8 (Fig.2), les unités de traitement 7 sont configurées pour déterminer la valeur significative de l'activité électrodermale $\mu_{AE,norm}$ en normalisant la valeur caractéristique de l'activité électrodermale AE$_c$ par rapport à une valeur caractéristique moyenne d'une activité électrodermale d'adaptation et de sa valeur correspondante d'écart-type (voir l'exemple de la Fig. 4).

La Fig. 4 illustre de manière schématique un schéma en bloc de surveillance de stress d'un utilisateur, selon un mode de réalisation préféré de l'invention.

Au bloc B1, le capteur 3 de rythme cardiaque est configuré pour mesurer au repos le rythme cardiaque de l'utilisateur pendant un intervalle de calibration $\Delta t_c$ prédéterminé. De même, le capteur 5 d'activité électrodermale est configuré pour mesurer l'activité électrodermale au repos de l'utilisateur pendant le même intervalle de calibration prédéterminé. Ces mesures sont ainsi réalisées pendant que l'utilisateur est au repos durant l'intervalle de calibration $\Delta t_c$ qui est avantageusement de l'ordre de 5 minutes (300 secondes). Bien entendu, la durée de cet intervalle peut être différente. Par exemple, cette durée de calibration peut avoir une valeur quelconque supérieure à 60 secondes sachant que plus la durée de calibration est grande, meilleure est la robustesse des mesures. Eventuellement, la durée de l'intervalle de calibration peut même être plus grande que 300 secondes.

Au bloc B2, les unités de traitement 7 sont configurées pour acquérir les mesures au repos du rythme cardiaque et de l'activité électrodermale depuis les capteurs. Ensuite, elles génèrent sur l'intervalle de calibration $\Delta t_c$ un signal de rythme cardiaque d'adaptation RC$_a$ ainsi qu'un signal d'activité électrodermale d'adaptation AE$_a$.

Au bloc B3, les unités de traitement 7 sont configurées pour calculer la valeur moyenne du rythme cardiaque d'adaptation $\mu_{RC,ref}$ et de sa valeur correspondante d'écart-type $\Delta\mu_{RC}$ à partir du signal du rythme cardiaque au repos.

La valeur moyenne du rythme cardiaque d'adaptation (ou de référence) $\mu_{RC,ref}$ est calculée en fonction du rythme cardiaque d'adaptation RC$_a$ selon la formule suivante :

$$\mu_{RC,ref} = \frac{1}{M} \sum_{k=1}^{M} RC_a(k)$$

avec M = $\Delta t_c$ /$\Delta t$

Avantageusement, l'intervalle de calibration $\Delta t_c$ est supérieur à l'intervalle opératoire $\Delta t$ (par exemple, $M \geq 3$)

Par ailleurs, l'écart-type $\Delta\mu_{RC}$ correspondant au rythme cardiaque d'adaptation est calculé selon la formule suivante :

$$\Delta\mu_{RC} = \sqrt{\sum_{k=1}^{M} \frac{1}{M} \left(RC_a(k) - \mu_{RC,ref}\right)^2}$$

Les unités de traitement 7 enregistrent la valeur moyenne du rythme cardiaque d'adaptation $\mu_{RC,ref}$ ainsi que l'écart-type correspondant $\Delta\mu_{RC}$.

Au bloc B4, le signal d'activité électrodermale au repos $AE_a$ est filtré par le filtre passe-bas d'ordre $N_f$ et de fréquence de coupure $f_c$ compris dans les unités de traitement 7 pour former un signal d'activité électrodermale d'adaptation filtré $AE_{a,filt}$.

Au bloc B5, les unités de traitement 7 sont configurées pour déterminer une valeur caractéristique d'activité électrodermale d'adaptation $AE_r$ sur l'intervalle de calibration en comptant le nombre de maxima dans le signal d'activité électrodermale filtré au repos. La valeur caractéristique d'activité électrodermale d'adaptation $AE_r$ est ainsi égale au nombre de maxima trouvés sur l'intervalle opératoire courant. La technique de détection de maxima est similaire à celle décrite en référence à la Fig.3.

Au bloc B6, les unités de traitement 7 sont configurées pour calculer la valeur caractéristique moyenne d'activité électrodermale d'adaptation $\mu_{AE,ref}$ et de sa valeur correspondante d'écart-type $\Delta\mu_{AE}$. Autrement dit, les unités de traitement 7 calculent le nombre moyen de maxima de l'activité électrodermale d'adaptation sur un intervalle égal à celui de l'intervalle opératoire courant.

La valeur caractéristique moyenne d'activité électrodermale d'adaptation (ou de référence) $\mu_{AE,ref}$ est calculée en fonction de la valeur caractéristique d'activité électrodermale d'adaptation $AE_r$ selon la formule suivante :

$$\mu_{AE,ref} = \frac{1}{M} \sum_{k=1}^{M} AE_r\,(k)$$

avec $M = \Delta t_c / \Delta t$ qui représente le nombre d'intervalles opératoires dans l'intervalle de calibration.

L'écart-type $\Delta\mu_{AE}$ correspondant au rythme cardiaque d'adaptation est calculé selon la formule suivante :

$$\Delta\mu_{AE} = \sqrt{\sum_{k=1}^{M} \frac{1}{M} \left(AE_r\,(k) - \mu_{AE,ref}\right)^2}$$

Les unités de traitement 7 enregistrent la valeur caractéristique moyenne d'activité électrodermale d'adaptation $\mu_{AE,ref}$ et l'écart-type correspondant $\Delta\mu_{RC}$.

Au bloc B11, le capteur 3 de rythme cardiaque est configuré pour mesurer en continu le rythme cardiaque de l'utilisateur portant le dispositif portable 10 ou 20. Simultanément, le capteur 5 d'activité électrodermale est configuré pour mesurer en continu l'activité électrodermale de l'utilisateur.

Au bloc B12, les unités de traitement 7 sont configurées pour acquérir les mesures du rythme cardiaque et de l'activité électrodermale depuis les capteurs 3 et 5. Ensuite, elles génèrent sur l'intervalle opérationnel $\Delta t$ courant (par exemple une fenêtre temporelle de 60 secondes) un signal du rythme cardiaque $S_{RC}$ ainsi qu'un signal d'activité électrodermale $S_{AE}$.

Au bloc B13, les unités de traitement 7 sont configurées pour échantillonner le signal courant du rythme cardiaque en formant un vecteur du rythme cardiaque $RC_f$ constitué de N échantillons sur l'intervalle opératoire courant. Simultanément, les unités de traitement 7 sont configurées pour échantillonner le signal courant d'activité électrodermale en formant un vecteur d'activité électrodermale $AE_f$ constitué de N échantillons sur l'intervalle opératoire courant.

Au bloc B14, les unités de traitement 7 sont configurées pour déterminer une valeur de rythme cardiaque moyen $\mu_{RC}$ sur l'intervalle opératoire courant (voir par exemple l'étape E3 de la Fig. 2).

Au bloc B15, le filtre passe-bas est configuré pour filtrer le signal d'activité électrodermale $AE_f$ formant un signal d'activité électrodermale filtré $AE_{filt}$ (voir l'exemple à l'étape E6 de la Fig. 2).

Au bloc B16, les unités de traitement 7 sont configurées pour déterminer une valeur caractéristique d'activité électrodermale $AE_c$ en comptant le nombre de maxima du signal d'activité électrodermale filtré $AE_{filt}$ sur l'intervalle opératoire courant (voir l'exemple à l'étape E7 de la Fig. 2).

Au bloc B17, les unités de traitement 7 sont configurées pour récupérer la valeur moyenne du rythme cardiaque d'adaptation $\mu_{RC,ref}$ et de sa valeur correspondante d'écart-type $\Delta\mu_{RC}$ préalablement enregistrées afin de déterminer la valeur significative de rythme cardiaque $\mu_{RC,norm}$ en normalisant la valeur de rythme cardiaque moyen $\mu_{RC}$ par rapport aux valeurs d'adaptation $\mu_{RC,ref}$ et $\Delta\mu_{RC}$. Cette normalisation peut être réalisée selon l'expression suivante :

$$\mu_{RC,norm} = \frac{\mu_{RC} - \mu_{RC,ref}}{\Delta\mu_{RC}}$$

Au bloc B18, les unités de traitement 7 sont configurées pour récupérer la valeur caractéristique moyenne $\mu_{AE,ref}$ de l'activité électrodermale d'adaptation et de sa valeur correspondante d'écart-type $\Delta\mu_{AE}$ préalablement enregistrées pour déterminer la valeur significative d'activité électrodermale $\mu_{AE,norm}$ en normalisant la valeur caractéristique d'activité électrodermale $AE_c$ par rapport aux valeurs d'adaptation $\mu_{AE,ref}$ et $\Delta\mu_{RC}$. Cette normalisation peut être réalisée selon l'expression suivante :

$$\mu_{AE,norm} = \frac{AE_c - \mu_{AE,ref}}{\Delta\mu_{AE}}$$

On notera que dans le cas où aucune calibration n'est faite, le procédé est toujours valable mais avec des valeurs de normalisation par défaut enregistrées préalablement dans les unités de traitement 7 . A titre d'exemple, la valeur moyenne du rythme cardiaque d'adaptation et sa valeur correspondante d'écart-type sont par défaut égales à 0 et 1 respectivement ($\mu_{RC,ref}$ = 0 et $\Delta\mu_{RC}$ = 1). De même, la valeur moyenne d'activité électrodermale d'adaptation et sa valeur correspondante d'écart-type sont par défaut égales à 0 et 1 respectivement ($\mu_{AE,ref}$ = 0 et $\Delta\mu_{AE}$ = 1).

Au bloc B19, les unités de traitement 7 sont configurées pour comparer la valeur significative de rythme cardiaque $\mu_{RC,norm}$ à un premier seuil prédéterminé $s_1$ (par exemple, $s_1$ = 1,5). Les unités de traitement 7 sont également configurées pour comparer la valeur significative d'activité électrodermale $\mu_{AE,norm}$ à un deuxième seuil prédéterminé $s_2$ (par exemple, $s_2$ = 2).

On notera que le premier seuil prédéterminé peut être égal à une valeur sélectionnée dans un intervalle compris entre 1 et 2. Par ailleurs, le deuxième seuil prédéterminé peut être égal à une valeur sélectionnée dans un intervalle compris entre 1,5 et 2,5.

Après la comparaison des valeurs significatives $\mu_{RC,norm}$ et $\mu_{AE,norm}$ aux premier et deuxième seuils $s_1$ et $s_2$ respectivement, les unités de traitement 7 sont configurées pour identifier un état de stress de l'utilisateur si les valeurs significatives de rythme cardiaque et d'activité électrodermale $\mu_{RC,norm}$ et $\mu_{AE,norm}$ dépassent simultanément et respectivement les premier et deuxième seuils prédéterminés $s_1$ et $s_2$. Ainsi l'utilisateur est considéré dans un état de stress lorsque la conjonction des inéquations suivantes est vérifiée :

$$\mu_{RC,norm} > s_1 \quad \text{et} \quad \mu_{AE,norm} > s_2$$

Au bloc B20, les unités de traitement 7 sont configurées pour envoyer en continu les données concernant l'état de stress S de l'utilisateur à l'interface 9. Cette dernière affiche cet état S sur un écran.

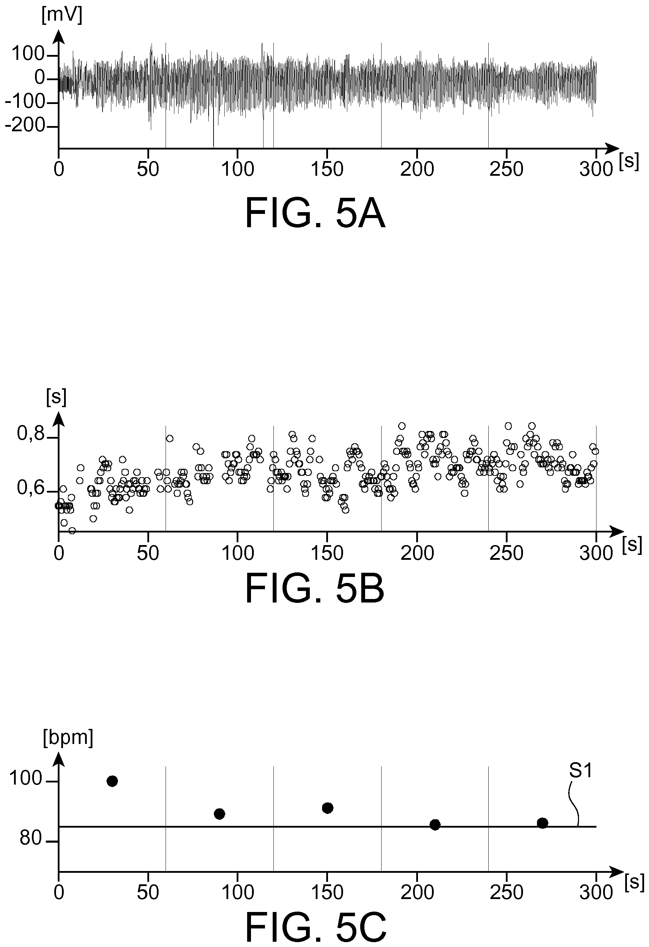

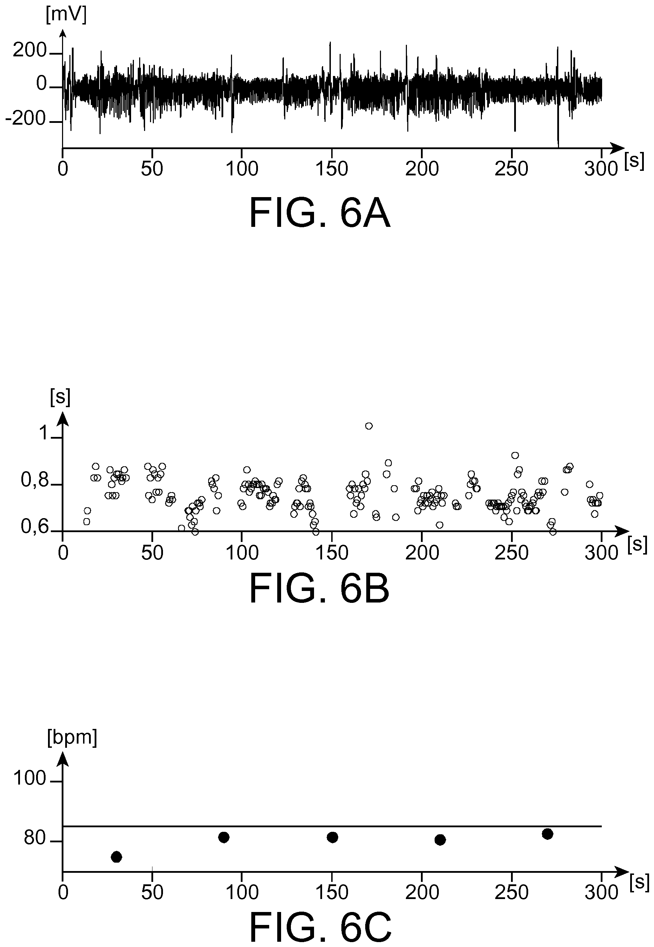

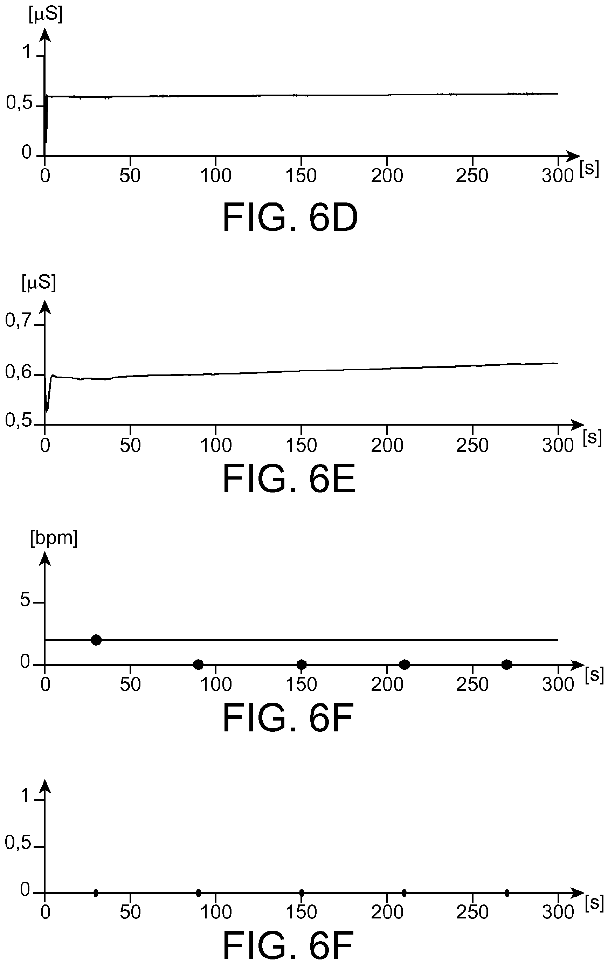

Les Figs. 5A-5G et 6A-6G sont des exemples de courbes illustrant les étapes de détection de l'état de stress d'un utilisateur.

Plus particulièrement, les Figs. 5A-5G montrent un exemple de détection d'un état de stress tandis que les Figs. 6A-6G montrent un exemple de détection d'un état de non stress de l'utilisateur.

La Fig. 5A montre la tension en mV du signal de rythme cardiaque brut en fonction du temps en secondes. L'intervalle temporel entre les lignes verticales représente l'intervalle opératoire qui est égal ici à 60 secondes.

La Fig. 5B montre le tachogramme correspondant au signal de rythme cardiaque de la Fig. 5A sur l'ensemble des intervalles opératoires.

La Fig. 5C montre la valeur significative de rythme cardiaque $\mu_{RC,norm}$ en battement par minute bpm sur chaque intervalle opératoire. La ligne horizontale représente le premier seuil $s_1$ de rythme cardiaque qui est égal ici à environ 1,4 (correspondant à environ 85 bpm). Dans cet exemple, on voit que la valeur sur chaque intervalle est supérieure au premier seuil $s_1$. On notera que cette figure peut également représenter le rythme cardiaque moyen $\mu_{RC}$ au cas où aucune normalisation n'a été réalisée.

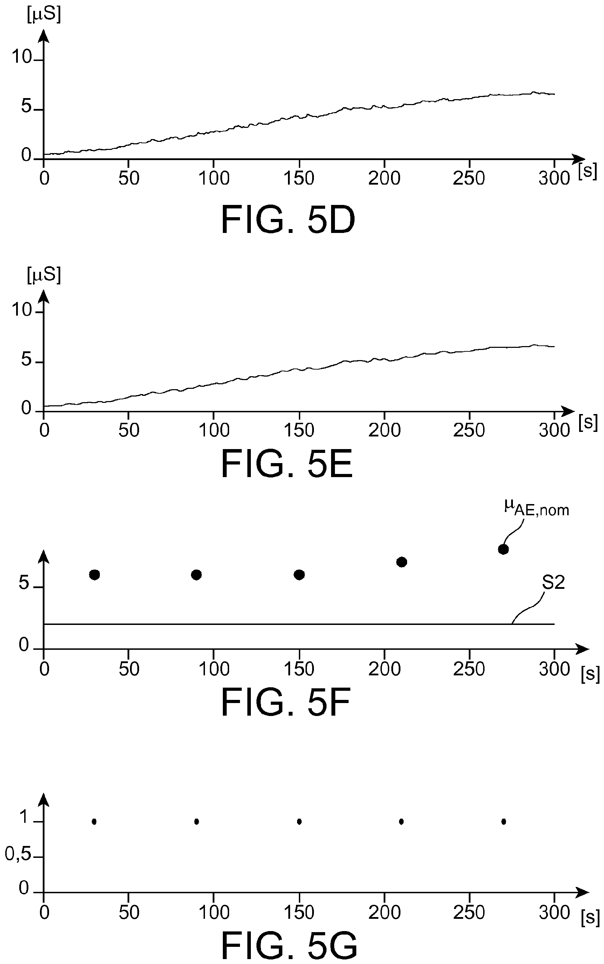

La Fig. 5D montre la conductance en $\mu$S du signal d'activité électrodermale brut en fonction du temps en secondes. De même, l'intervalle temporel entre les lignes verticales représente l'intervalle opératoire qui est aussi égal à 60 secondes.

La Fig. 5E montre le signal d'activité électrodermale filtré $AE_{filt}$.

La Fig. 5F montre la valeur significative d'activité électrodermale $\mu_{AE,norm}$ représentant un nombre significatif de maxima sur chaque intervalle opératoire. La ligne horizontale représente le deuxième seuil $s_2$ d'activité électrodermale qui est égal ici à 2 ($s_2$ = 2). Dans cet exemple, on voit que la valeur sur chaque intervalle est supérieure au deuxième seuil $s_2$. On notera que cette figure peut également représenter la valeur caractéristique d'activité électrodermale $AE_c$ au cas où aucune normalisation n'a été réalisée.

La Fig. 5G montre le repérage d'un état de stress de l'utilisateur sur l'ensemble des intervalles opératoires car les valeurs significatives de rythme cardiaque et d'activité électrodermale $\mu_{RC,norm}$ et $\mu_{AE,norm}$ dépassent simultanément et respectivement les premier et deuxième seuils prédéterminés $s_1$ et $s_2$. Un état de stress est représenté par la valeur S=1 et un état de non stress par la valeur S=0.

Les Figs. 6A-6G sont similaires à celles des Figs. 5A-5G à part le fait que les valeurs significatives de rythme cardiaque et d'activité électrodermale $\mu_{RC,norm}$ et $\mu_{AE,norm}$ ne dépassent pas simultanément les premier et deuxième seuils prédéterminés $s_1$ et $s_2$ sur aucun intervalle opératoire et par conséquent, l'utilisateur n'est pas dans un état de stress sur l'ensemble des intervalles opératoires.

Avantageusement, les unités de traitement 7 sont configurées pour évaluer le niveau de stress en fonction des écarts entre d'une part les valeurs significatives de rythme cardiaque et d'activité électrodermale et d'autre part les premier et deuxième seuils prédéterminés. Dans ce cas, l'état de stress est affecté d'un certain poids qui dépend de l'écart entre les valeurs significatives et les seuils correspondants.

En variante, on peut définir un premier seuil d'un premier niveau et un premier seuil d'un deuxième niveau et de même, on peut définir un deuxième seuil d'un premier niveau et un deuxième seuil d'un deuxième niveau. Ainsi, si les valeurs significatives de rythme cardiaque et d'activité électrodermale $\mu_{RC,norm}$ et $\mu_{AE,norm}$ dépassent simultanément les premier et deuxième seuils du deuxième niveau, le dispositif identifie alors un état de stress élevé.

## Revendications

1. Procédé non intrusif de surveillance de stress d'un utilisateur, ledit procédé étant **caractérisé en ce qu'**il comporte les étapes suivantes :

   - mesure en continu d'un rythme cardiaque dudit utilisateur,
   - mesure en continu d'une activité électrodermale dudit utilisateur,
   - extraction d'une valeur significative de rythme cardiaque sur un intervalle opératoire courant de temps prédéterminé,
   - extraction d'une valeur significative d'activité électrodermale sur ledit intervalle opératoire courant, ladite extraction comprenant une détermination d'une valeur caractéristique d'activité électrodermale en comptant un nombre de maxima dudit signal d'activité électrodermale sur ledit intervalle opératoire courant,
   - comparaison de ladite valeur significative de rythme cardiaque à un premier seuil prédéterminé,
   - comparaison de ladite valeur significative d'activité électrodermale à un deuxième seuil prédéterminé, et
   - identification d'un état de stress dudit utilisateur lorsque lesdites valeurs significatives de rythme cardiaque et d'activité électrodermale dépassent simultanément et respectivement les premier et deuxième seuils prédéterminés.

2. Procédé selon la revendication 1, **caractérisé en ce que** l'extraction de ladite valeur significative de rythme cardiaque comporte les étapes suivantes :

   - génération d'un signal du rythme cardiaque représentatif de ladite mesure du rythme cardiaque sur ledit intervalle opératoire courant,
   - détermination à partir dudit signal du rythme cardiaque d'une valeur de rythme cardiaque moyen sur ledit intervalle opératoire courant, et
   - détermination de ladite valeur significative de rythme cardiaque en normalisant la valeur de rythme cardiaque moyen par rapport à une valeur moyenne d'un rythme cardiaque d'adaptation et de sa valeur correspondante d'écart-type.

3. Procédé selon la revendication 1, **caractérisé en ce que** l'extraction de ladite valeur significative d'activité électrodermale comporte :

   - une génération d'un signal d'activité électrodermale représentatif de ladite mesure de l'activité électrodermale sur ledit intervalle opératoire courant, et
   - détermination de ladite valeur significative d'activité électrodermale en normalisant ladite valeur caractéristique d'activité électrodermale par rapport à une valeur caractéristique moyenne d'une activité électrodermale d'adaptation et de sa valeur correspondante d'écart-type.

4. Procédé selon l'une quelconque des revendications 1 à 3, **caractérisé en ce qu'**il comporte un filtrage dudit signal d'activité électrodermale avant la détermination de ladite valeur caractéristique d'activité électrodermale.

5. Procédé selon la revendication 2 ou selon l'une quelconque des revendications 3 à 4 lorsque la revendication est dépendante de la revendication 2, **caractérisé en ce que** la détermination de ladite valeur moyenne de rythme cardiaque d'adaptation et de sa valeur correspondante d'écart-type comporte :

- une mesure d'un rythme cardiaque au repos dudit utilisateur pendant un intervalle de calibration prédéterminé,
- une génération d'un signal du rythme cardiaque au repos sur ledit intervalle de calibration, et
- un calcul de ladite valeur moyenne de rythme cardiaque d'adaptation et de sa valeur correspondante d'écart-type.

6.  Procédé selon la revendication 3 ou selon la revendication 4 lorsque la revendication est dépendante de la revendication 3, **caractérisé en ce que** la détermination de ladite valeur caractéristique moyenne d'activité électrodermale d'adaptation et de ladite valeur correspondante d'écart-type comporte :

- une mesure d'une activité électrodermale au repos dudit utilisateur pendant un intervalle de calibration prédéterminé,
- une génération d'un signal d'activité électrodermale au repos sur ledit intervalle de calibration,
- une détermination de ladite valeur caractéristique d'activité électrodermale d'adaptation sur ledit intervalle de calibration en comptant un nombre de maxima dudit signal d'activité électrodermale au repos, et
- un calcul de ladite valeur caractéristique moyenne d'activité électrodermale d'adaptation et de sa valeur correspondante d'écart-type.

7.  Procédé selon les revendications 3 et 5, **caractérisé en ce que** ladite valeur moyenne d'un rythme cardiaque d'adaptation et sa valeur correspondante d'écart-type sont par défaut égales à « 0 » et « 1 » respectivement, et **en ce que** ladite valeur moyenne d'activité électrodermale d'adaptation et la valeur correspondante d'écart-type sont par défaut égales à « 0 » et « 1 » respectivement.

8.  Procédé selon l'une quelconque des revendications précédentes, **caractérisé en ce qu'**il comporte une évaluation d'un niveau de stress en fonction des écarts entre d'une part lesdites valeurs significatives de rythme cardiaque et d'activité électrodermale et d'autre part les premier et deuxième seuils prédéterminés.

9.  Procédé selon l'une quelconque des revendications précédentes, **caractérisé en ce que** le premier seuil prédéterminé correspond à une valeur sélectionnée dans un intervalle compris entre 1 et 2, et **en ce que** le deuxième seuil prédéterminé correspond à une valeur sélectionnée dans un intervalle compris entre 1,5 et 2,5.

10. Procédé selon l'une quelconque des revendications précédentes, **caractérisé en ce que** l'intervalle opératoire courant de temps prédéterminé est une période dont la valeur est comprise entre environ 15 secondes et environ 300 secondes.

11. Procédé selon la revendication 5 ou 6 ou selon l'une quelconque des revendications 7 à 10 lorsque la revendication est dépendante de la revendication 5 ou 6, **caractérisé en ce que** l'intervalle de calibration prédéterminé est une période dont la valeur est plus grande ou égale à environ 300 secondes.

12. Système non intrusif de surveillance en continu de l'état de stress d'un utilisateur, **caractérisé en ce qu'**il comporte :

- un capteur (3) de rythme cardiaque configuré pour mesurer en continu un rythme cardiaque dudit utilisateur,
- un capteur (5) d'activité électrodermale configuré pour mesurer en continu une activité électrodermale dudit utilisateur, et
- des unités de traitement (7) configurées pour :

- extraire une valeur significative de rythme cardiaque sur un intervalle opératoire courant de temps prédéterminé,
- extraire une valeur significative d'activité électrodermale sur ledit intervalle opératoire courant, ladite extraction comprenant une détermination d'une valeur caractéristique d'activité électrodermale en comptant un nombre de maxima dudit signal d'activité électrodermale sur ledit intervalle opératoire courant,
- comparer ladite valeur significative de rythme cardiaque à un premier seuil prédéterminé,
- comparer ladite valeur significative d'activité électrodermale à un deuxième seuil prédéterminé, et
- identifier un état de stress dudit utilisateur lorsque lesdites valeurs significatives de rythme cardiaque et d'activité électrodermale dépassent simultanément et respectivement les premier et deuxième seuils prédéterminés.

**13.** Système selon la revendication 12, **caractérisé en ce que** les unités de traitement (7) sont configurées (7) pour extraire ladite valeur significative de rythme cardiaque en :

- générant un signal de rythme cardiaque représentatif de ladite mesure du rythme cardiaque sur ledit intervalle opératoire courant,
- déterminant une valeur de rythme cardiaque moyen sur ledit intervalle opératoire courant, - déterminant ladite valeur significative de rythme cardiaque par normalisation de la valeur de rythme cardiaque moyen par rapport à une valeur moyenne d'un rythme cardiaque d'adaptation et de sa valeur correspondante d'écart-type,

et **en ce que** les unités de traitement (7) sont configurées pour extraire ladite valeur significative d'activité électrodermale en :

- générant un signal d'activité électrodermale représentatif de ladite mesure de l'activité électrodermale sur ledit intervalle opératoire courant,
- déterminant une valeur caractéristique d'activité électrodermale par un comptage d'un nombre de maxima dudit signal d'activité électrodermale sur ledit intervalle opératoire courant, et
- déterminant ladite valeur significative d'activité électrodermale par normalisation de ladite valeur caractéristique d'activité électrodermale par rapport à une valeur caractéristique moyenne d'une activité électrodermale d'adaptation et de sa valeur correspondante d'écart-type.

**14.** Système selon la revendication 12 ou 13, **caractérisé en ce qu'**il comporte une interface (9) indiquant l'état de stress de l'utilisateur.

**15.** Bracelet comportant le système selon l'une quelconque des revendications 12 à 14.


**Patentansprüche**

**1.** Nicht-invasives Verfahren zur Überwachung des Stresses eines Benutzers, wobei das Verfahren **dadurch gekennzeichnet ist, dass** es die folgenden Schritte aufweist:

- kontinuierliches Messen eines Herzrhythmus des genannten Nutzers,
- kontinuierliches Messen einer elektrodermalen Aktivität des genannten Nutzers,
- Extrahieren eines signifikanten Herzfrequenzwerts über ein momentanes, vorbestimmtes Zeitintervall,
- Extrahieren eines signifikanten Wertes der elektrodermalen Aktivität über das momentane Betriebsintervall, wobei das Extrahieren ein Bestimmen eines charakteristischen Wertes der elektrodermalen Aktivität durch Zählen einer Anzahl von Maxima des Signals der elektrodermalen Aktivität über das momentane Betriebsintervall aufweist,
- Vergleichen des vorgenannten signifikanten Herzfrequenzwerts mit einem ersten vorbestimmten Schwellenwert,
- Vergleichen des vorgenannten signifikanten Werts der elektrodermalen Aktivität mit einem zweiten vorbestimmten Schwellenwert und
- Identifizieren eines Stresszustands des Benutzers, wenn die signifikanten Werte der Herzfrequenz und der elektrodermalen Aktivität gleichzeitig jeweils den ersten und zweiten vorbestimmten Schwellenwert überschreiten.

**2.** Verfahren nach Anspruch 1, **dadurch gekennzeichnet, dass** das Extrahieren des vorgenannten signifikanten Wertes der Herzfrequenz die folgenden Schritte aufweist:

- Erzeugen eines Herzfrequenzsignals, das für die Herzfrequenzmessung über das momentane Betriebsintervall repräsentativ ist,
- Bestimmen eines Wertes für die durchschnittliche Herzfrequenz über das momentane Betriebsintervall aus dem Herzfrequenzsignal; und
- Bestimmen des vorgenannten signifikanten Werts der Herzfrequenz durch Normalisieren des durchschnittlichen Herzfrequenzwerts in Bezug auf einen Durchschnittswert einer ausgebildeten Herzfrequenz und deren entsprechenden Wert der Standardabweichung.

**3.** Verfahren nach Anspruch 1, **dadurch gekennzeichnet, dass** das Extrahieren des vorgenannten signifikanten Wertes der elektrodermalen Aktivität folgendes aufweist:

- Erzeugen eines elektrodermalen Aktivitätssignals, das für die Messung der elektrodermalen Aktivität über das momentane Betriebsintervall repräsentativ ist, und
- Bestimmen des signifikanten Wertes der elektrodermalen Aktivität durch Normalisieren des charakteristischen Wertes der elektrodermalen Aktivität auf einen durchschnittlichen charakteristischen Wert einer ausgebildeten elektrodermalen Aktivität und deren entsprechenden Wert der Standardabweichung.

4. Verfahren nach einem der Ansprüche 1 bis 3, **gekennzeichnet durch** eine Filterung des Signals der elektrodermalen Aktivität vor der Bestimmung des charakteristischen Wertes der elektrodermalen Aktivität.

5. Verfahren nach Anspruch 2 oder nach einem der Ansprüche 3 bis 4, soweit der Anspruch von Anspruch 2 abhängig ist, **dadurch gekennzeichnet, dass** die Bestimmung des Mittelwerts der derart ausgebildeten Herzfrequenz und ihres entsprechenden Werts der Standardabweichung folgendes aufweist:

- Messen einer Ruheherzfrequenz des genannten Benutzers während eines vorbestimmten Kalibrierungsintervalls,
- Erzeugen eines Signals der Ruheherzfrequenz über das genannte Kalibrierungsintervall, und
- Berechnen des derart ausgebildeten Herzfrequenzmittelwerts und seines entsprechenden Werts für die Standardabweichung.

6. Verfahren nach Anspruch 3 oder nach Anspruch 4, soweit der Anspruch von Anspruch 3 abhängig ist, **dadurch gekennzeichnet, dass** die Bestimmung des charakteristischen Mittelwertes der ausgebildeten elektrodermalen Aktivität und des entsprechenden Wertes der Standardabweichung folgendes aufweist:

- Messen der elektrodermalen Aktivität im Ruhezustand des genannten Benutzers während eines vorbestimmten Kalibrierungsintervalls,
- Erzeugen eines Signals der elektrodermalen Aktivität im Ruhezustand über das Kalibrierungsintervall,
- Bestimmen des derart ausgebildeten elektrodermalen Aktivitätskennwerts über das Kalibrierungsintervall durch Zählen einer Anzahl von Maxima des elektrodermalen Aktivitätssignals im Ruhezustand, und
- Berechnen des derart ausgebildeten charakteristischen Mittelwerts der elektrodermalen Aktivität und seines entsprechenden Werts der Standardabweichung.

7. Verfahren nach Anspruch 3 und 5, **dadurch gekennzeichnet, dass** der Mittelwert einer derart ausgebildeten Herzfrequenz und sein entsprechender Wert für die Standardabweichung standardmäßig jeweils "0" und "1" betragen, und dass der derart ausgebildete Durchschnittswert der elektrodermalen Aktivität und der entsprechende Wert der Standardabweichung standardmäßig jeweils "0" und "1" betragen.

8. Verfahren nach einem der vorhergehenden Ansprüche, **gekennzeichnet durch** eine Bewertung eines Stressniveaus in Abhängigkeit von den Abweichungen zwischen den signifikanten Werten des Herzrhythmus und der elektrodermalen Aktivität einerseits und dem ersten und dem zweiten vorbestimmten Schwellenwert andererseits.

9. Verfahren nach einem der vorhergehenden Ansprüche, **dadurch gekennzeichnet, dass** der erste vorbestimmte Schwellenwert einem Wert entspricht, der aus einem Bereich zwischen 1 und 2 ausgewählt ist, und dass der zweite vorbestimmte Schwellenwert einem Wert entspricht, der aus einem Bereich zwischen 1,5 und 2,5 ausgewählt ist.

10. Verfahren nach einem der vorhergehenden Ansprüche, **dadurch gekennzeichnet, dass** das momentane Betriebsintervall der vorbestimmten Zeit eine Periode ist, deren Wert zwischen etwa 15 Sekunden und etwa 300 Sekunden liegt.

11. Verfahren nach Anspruch 5 oder 6 oder nach einem der Ansprüche 7 bis 10, soweit der Anspruch von Anspruch 5 oder 6 abhängt, **dadurch gekennzeichnet, dass** das vorbestimmte Kalibrierungsintervall eine Periode ist, deren Wert größer oder gleich etwa 300 Sekunden ist.

12. Nicht-invasives System zur kontinuierlichen Überwachung des Stresszustands eines Benutzers, **dadurch gekennzeichnet, dass** es folgendes aufweist:

- einen Herzfrequenzsensor (3), welcher derart konfiguriert ist, dass er kontinuierlich eine Herzfrequenz des genannten Benutzers misst,

- einen Sensor (5) für elektrodermale Aktivität, welcher derart konfiguriert ist, dass er kontinuierlich eine elektrodermale Aktivität des Benutzers misst, und

- Verarbeitungseinheiten (7), welche konfiguriert sind zum:

- Extrahieren eines signifikanten Herzfrequenzwerts über ein momentanes, vorbestimmtes Betriebszeitintervall,

- Extrahieren eines signifikanten Wertes der elektrodermalen Aktivität über das momentane Betriebsintervall, wobei das Extrahieren das Bestimmen eines charakteristischen Wertes der elektrodermalen Aktivität durch Zählen einer Anzahl von Maxima des elektrodermalen Aktivitätssignals über das momentane Betriebsintervall aufweist,

- Vergleichen des vorgenannten signifikanten Werts der Herzfrequenz mit einem ersten vorbestimmten Schwellenwert,

- Vergleichen des vorgenannten signifikanten Wertes der elektrodermalen Aktivität mit einem zweiten vorbestimmten Schwellenwert zu und

- Identifizieren eines Stresszustands des Benutzers, falls die signifikanten Werte der Herzfrequenz und der elektrodermalen Aktivität gleichzeitig jeweils den ersten und den zweiten vorbestimmten Schwellenwert überschreiten.

13. System nach Anspruch 12, **dadurch gekennzeichnet, dass** die Verarbeitungseinheiten (7) derart konfiguriert sind (7), dass sie den signifikanten Wert der Herzfrequenz extrahieren mit:

- Erzeugen eines Herzfrequenzsignals, das für die Messung der Herzfrequenz über das momentane Betriebsintervall repräsentativ ist,

- Bestimmen eines Wertes der durchschnittlichen Herzfrequenz über das momentane Betriebsintervall,

- Bestimmen des vorgenannten signifikanten Wertes der Herzfrequenz durch Normalisieren des Wertes der durchschnittlichen Herzfrequenz in Bezug auf einen Durchschnittswert einer derart ausgebildeten Herzfrequenz und deren entsprechenden Wert der Standardabweichung,

- wobei die Verarbeitungseinheiten (7) derart konfiguriert sind, dass sie den vorgenannten signifikanten Wert der elektrodermalen Aktivität extrahieren mit:

- Erzeugen eines elektrodermalen Aktivitätssignals, das für die Messung der elektrodermalen Aktivität über das momentane Betriebsintervall repräsentativ ist,

- Bestimmen eines charakteristischen Werts der elektrodermalen Aktivität durch Zählen einer Anzahl von Maxima des Signals der elektrodermalen Aktivität über das momentane Betriebsintervall, und

- Bestimmen des signifikanten Wertes der elektrodermalen Aktivität durch Normalisieren des charakteristischen Wertes der elektrodermalen Aktivität auf einen durchschnittlichen charakteristischen Wert einer ausgebildeten elektrodermalen Aktivität und deren entsprechenden Wert der Standardabweichung.

14. System nach Anspruch 12 oder 13, **dadurch gekennzeichnet, dass** es eine Schnittstelle (9) aufweist, die den Stresszustand des Benutzers anzeigt.

15. Armband, das das System nach einem der Ansprüche 12 bis 14 aufweist.

## Claims

1. A non-intrusive method for monitoring a stress in a user, said method being **characterised in that** it includes the following steps:

- continuously measuring a heart rate of said user,

- continuously measuring an electrodermal activity of said user,

- extracting a significant heart rate value over a predetermined current operating time interval,

- extracting a significant electrodermal activity value over said current operating interval, said extraction comprising a determination of a characteristic value of electrodermal activity by counting a number of maxima of said electrodermal activity signal over said current operating interval,

- comparing said significant heart rate value to a first predetermined threshold,

- comparing said significant electrodermal activity value to a second predetermined threshold, and

- identifying a stress state of said user when said significant values of heart rate and electrodermal activity simultaneously and respectively exceed the first and second predetermined thresholds.

2. The method according to claim 1, **characterised in that** the extraction of said significant heart rate value includes the following steps:

   - generating a heart rate signal representative of said heart rate measurement over said current operating interval,
   - determining a mean heart rate value from said heart rate signal over said current operating interval, and
   - determining said significant heart rate value by normalising the mean heart rate value relative to a mean value of an adaptation heart rate and the corresponding standard deviation value thereof.

3. The method according to claim 1, **characterised in that** the extraction of said significant electrodermal activity value includes:

   - generating an electrodermal activity signal representative of said measurement of the electrodermal activity over said current operating interval, and
   - determining said significant electrodermal activity value by normalising said characteristic value of electrodermal activity relative to a mean characteristic value of an adaptation electrodermal activity and the corresponding standard deviation value thereof.

4. The method according to any one of claims 1 to 3, **characterised in that** it includes a filtering of said electrodermal activity signal before determining said characteristic value of electrodermal activity.

5. The method according to claim 2 or according to any one of claims 3 to 4 when the claim is dependent on claim 2, **characterised in that** the determination of said mean adaptation heart rate value and the corresponding standard deviation value thereof includes:

   - a measurement of a resting heart rate of said user during a predetermined calibration interval,
   - a generation of a resting heart rate signal over said calibration interval, and
   - a calculation of said mean adaptation heart rate value and the corresponding standard deviation value thereof.

6. The method according to claim 3 or according to claim 4 when the claim is dependent on claim 3, **characterised in that** the determination of said mean characteristic value of adaptation electrodermal activity and of said corresponding standard deviation value includes:

   - a measurement of a resting electrodermal activity of said user during a predetermined calibration interval,
   - a generation of a resting electrodermal activity signal over said calibration interval,
   - a determination of said characteristic value of adaptation electrodermal activity over said calibration interval by counting a number of maxima of said resting electrodermal activity signal, and
   - a calculation of said mean characteristic value of adaptation electrodermal activity and the corresponding standard deviation value thereof.

7. The method according to claims 3 and 5, **characterised in that** said mean value of an adaptation heart rate and the corresponding standard deviation value thereof are by default equal to "0" and "1" respectively, and **in that** said mean adaptation electrodermal activity value and the corresponding standard deviation value are by default equal to "0" and "1" respectively.

8. The method according to any one of the preceding claims, **characterised in that** it includes an evaluation of a level of stress depending on the differences between, on the one hand, said significant values of heart rate and electrodermal activity and, on the other hand, the first and second predetermined thresholds.

9. The method according to any one of the preceding claims, **characterised in that** the first predetermined threshold corresponds to a value selected within an interval comprised between 1 and 2, and **in that** the second predetermined threshold corresponds to a value selected within an interval comprised between 1.5 and 2.5.

10. The method according to any one of the preceding claims, **characterised in that** the predetermined current operating time interval is a period whose value is comprised between about 15 seconds and about 300 seconds.

11. The method according to claim 5 or 6 or according to any one of claims 7 to 10 when the claim is dependent on claim 5 or 6, **characterised in that** the predetermined calibration interval is a period whose value is greater than or equal to about 300 seconds.

12. A non-intrusive system for continuous monitoring of the stress state of a user, **characterised in that** it includes:

- a heart rate sensor (3) configured to continuously measure a heart rate of said user,
- an electrodermal activity sensor (5) configured to continuously measure an electrodermal activity of said user, and
- processing units (7) configured to:

- extract a significant heart rate value over a predetermined current operating time interval,
- extract a significant electrodermal activity value over said current operating interval, said extraction comprising a determination of a characteristic value of electrodermal activity by counting a number of maxima of said electrodermal activity signal over said current operating interval,
- compare said significant heart rate value to a first predetermined threshold,
- compare said significant electrodermal activity value to a second predetermined threshold, and
- identify a stress state of said user when said significant values of heart rate and electrodermal activity simultaneously and respectively exceed the first and second predetermined thresholds.

13. The system according to claim 12, **characterised in that** the processing units (7) are configured (7) to extract said significant heart rate value by:

- generating a heart rate signal representative of said heart rate measurement over said current operating interval,
- determining a mean heart rate value over said current operating interval,
- determining said significant heart rate value by normalising the mean heart rate value relative to a mean value of an adaptation heart rate and the corresponding standard deviation value thereof,

and **in that** the processing units (7) are configured to extract said significant electrodermal activity value by:

- generating an electrodermal activity signal representative of said measurement of the electrodermal activity over said current operating interval,
- determining a characteristic value of electrodermal activity by counting a number of maxima of said electrodermal activity signal over said current operating interval, and
- determining said significant electrodermal activity value by normalising said characteristic value of electrodermal activity relative to a mean characteristic value of an adaptation electrodermal activity and the corresponding standard deviation value thereof.

14. The system according to claim 12 or 13, **characterised in that** it includes an interface (9) indicating the stress state of the user.

15. A bracelet including the system according to any one of claims 12 to 14.

FIG. 1A

FIG. 1B

FIG. 1C

FIG. 2

FIG. 3

FIG. 4

EP 3 305 186 B1

FIG. 5A

FIG. 5B

FIG. 5C

FIG. 5D

FIG. 5E

FIG. 5F

FIG. 5G

**FIG. 6A**

**FIG. 6B**

**FIG. 6C**

FIG. 6D

FIG. 6E

FIG. 6F

FIG. 6F

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2016089038 A

- US 20160262690 A

**Littérature non-brevet citée dans la description**

- **GARCIA-MANCILLA et al.** Stress quantification using a wearable device for daily feedback to improve stress management. *Lecture Notes in Computer Science,* 2016, vol. 9545, 204-209

- **ALEXANDRATOS et al.** Mobile real-time arousal détection. *ICASSP,* 04 Mai 2014